(19) Europäisches Patentamt
European Patent Office
Office européen des brevets

(11) **EP 4 076 717 B1**

(12) **EUROPÄISCHE PATENTSCHRIFT**

(45) Veröffentlichungstag und Bekanntmachung des Hinweises auf die Patenterteilung:
**21.02.2024   Patentblatt 2024/08**

(21) Anmeldenummer: **19831658.0**

(22) Anmeldetag: **17.12.2019**

(51) Internationale Patentklassifikation (IPC):
**C09K 23/00** *(2022.01)*      **C09K 23/54** *(2022.01)*

(52) Gemeinsame Patentklassifikation (CPC):
**C09K 23/002; C09K 23/54**

(86) Internationale Anmeldenummer:
**PCT/EP2019/085651**

(87) Internationale Veröffentlichungsnummer:
**WO 2021/121562 (24.06.2021 Gazette 2021/25)**

(54) **KLEINTEILIGE WÄSSRIGE PARTIKELSTABILISIERTE PICKERING EMULSION UND DARAUS HERGESTELLTE PARTIKEL**

FINELY DIVIDED AQUEOUS PARTICLE-STABILIZED PICKERING EMULSION AND PARTICLES PRODUCED THEREFROM

ÉMULSION DE PICKERING AQUEUSE STABILISÉE PAR DES PARTICULES FINEMENT DIVISÉE ET PARTICULES PRODUITES À PARTIR DE CELLE-CI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.10.2022   Patentblatt 2022/43**

(73) Patentinhaber: **Wacker Chemie AG**
**81737 München (DE)**

(72) Erfinder: **KNÖR, Sebastian**
**84547 Emmerting (DE)**

(74) Vertreter: **Belz, Ferdinand et al**
**Wacker Chemie AG**
**Hanns-Seidel-Platz 4**
**81737 München (DE)**

(56) Entgegenhaltungen:
**EP-A1- 0 433 727          DE-A1- 19 742 761**
**DE-A1-102006 014 875     DE-A1-102015 216 415**
**US-A1- 2017 319 458**

Bemerkungen:
Die Akte enthält technische Angaben, die nach dem Eingang der Anmeldung eingereicht wurden und die nicht in dieser Patentschrift enthalten sind.

EP 4 076 717 B1

**Beschreibung**

Die Erfindung betrifft eine wässrige partikelstabilisierte Pickering Emulsion eines polyadditionsfähigen, polykondensationsfähigen oder polymerisierbaren Materials, ein Verfahren zu deren Herstellung, ein Verfahren zur Herstellung von Partikeln aus dieser Pickering Emulsion und Partikel.

WO2017142068A1 offenbart feinteilige Silica-stabilisierte Emulsionen von reaktiven, additionsvernetzenden Siloxanen unter nachteiliger Verwendung von sehr großen Mengen an Silica. Der Kern der Siliconelastomer-Partikel wird über eine Polyadditionsreaktion gebildet.

Auch WO2007113095 beschreibt die Herstellung von Siliconpartikeln über Silica-stabilisierte Emulsionen. Es wird gelehrt, dass die Größe der Partikel sich zum Beispiel durch die Emulgiertechnik, also etwa durch Größen wie die eingetragene Scherenergie, den Volumenanteil der dispersen siliciumorganischen Phase, die Menge der stabilisierenden feinteiligen Metalloxidpartikel, den pH-Wert der kontinuierlichen Wasserphase und deren Ionenstärke, die Viskosität, die Reihenfolge der Dosierung, die Dosiergeschwindigkeit, oder durch die Reaktionsführung steuern lässt, das heißt zum Beispiel durch die Reaktionstemperatur, die Reaktionszeit, die Konzentrationen der eingesetzten Rohstoffe. Die Auswahl und die Menge des gegebenenfalls eingesetzten Hydrolyse- und Kondensationskatalysators hat ebenfalls einen Einfluss auf die Partikelgröße. WO2007113095 lehrt außerdem, dass man bei Verwendung einer Emulgiertechnik, die die Herstellung kleinerer Tröpfchen erlaubt, nach diesem Verfahren zu kleinen oberflächenstrukturierten Partikeln gelangt. Hierzu kann man beispielsweise andere Scherenergien oder eine Auswahl anderer amphiphiler Partikel zur Stabilisierung der kondensationsfähigen Flüssigkeit oder Zubereitung in Wasser verwenden.

Gegenstand der vorliegenden Erfindung ist ein Verfahren zur Herstellung einer wässrigen partikelstabilisierten Pickering Emulsion (E) eines polyadditionsfähigen, polykondensationsfähigen oder polymerisierbaren Materials (S), das ausgewählt wird aus Siloxan und Silan,

bei dem eine wässrige Phase (W),
ein polyadditionsfähiges, polykondensationsfähiges oder polymerisierbares Material (S), das ausgewählt wird aus Siloxan und Silan und
ein partikulärer Silicium(IV)oxid Feststoff (F) vermischt werden,
wobei Tröpfchen mit einem mittleren Durchmesser $d_{50}$ von höchstens 9$\mu$m gebildet werden, welche das Material (S) und den partikulären Feststoff (F) umfassen,
mit der Maßgabe, dass in der partikelstabilisierten Pickering-Emulsion
das Massenverhältnis Q1 = m(F)/m(S)*100 eine beliebige Zahl von 3 bis 25 ist,
das Massenverhältnis Q2 = m(S)/(m(S)+m(W))*100 eine beliebige Zahl von 50 bis 75 ist und
Q1 und Q2 zueinander im Zusammenhang

$$Q2 = -(1,56 *Q1) + Q3$$

stehen, wobei
Q3 eine Zahl zwischen 77,0 und 89,0 ist.

Überraschend wurde gefunden, dass die kleinteiligen wässrigen partikelstabilisierten Pickering-Emulsionen (E) gebildet werden, wenn die vorstehend beschriebenen Massenverhältnisse eingehalten werden. Solche kleinteiligen Emulsionen (E) sind sonst nur bei Verwendung von organischen Emulgatoren zugänglich, welche störend den Tröpfchen anhaften. Weitere kleinteilige, beschichtete Partikel sind aus EP433727, DE 102006014875 und DE 102015216415 bekannt.

Die Größe der Tröpfchen mit einem mittleren Durchmesser $d_{50}$ kleiner 9$\mu$m ist im erfindungsgemäßen Verfahren weitgehend unabhängig vom Mischverfahren und der Mischenergie. Damit ist das erfindungsgemäße Verfahren wesentlich robuster und ermöglicht eine Veränderung der Ansatzgröße oder des Mischverfahrens, wie beispielsweise beim betrieblichen Scaleup, ohne dass eine aufwändige Anpassung der Rezeptur oder des Verfahrens erforderlich ist.

Die Pickering-Emulsionen (E) haben gegenüber den nicht-erfindungsgemäßen grobteiligen Emulsionen nach dem Stand der Technik den Vorteil, dass sie eine verbesserte Stabilität gegen Separation haben, das heißt weitgehend stabil gegen Aufrahmen oder Sedimentation der dispersen Phase sind.

Vorzugsweise zeigt die Pickering Emulsion E innerhalb von 21 Tagen Lagerung bei Raumtemperatur keine nennenswerte Separation.

Die Pickering-Emulsionen (E) haben weiter den Vorteil, dass sie niedrige Scherviskositäten aufweisen und somit eine leichte Applikation ermöglichen. Die erfindungsgemäßen wässrigen Emulsionen haben des Weiteren den Vorteil, dass sie eine hohe Lagerstabilität in einem geschlossenen System bei Raumtemperatur von mindestens 15 Monaten aufweisen.

Die Pickering-Emulsionen (E) haben ferner den Vorteil, dass auf organische Emulgatoren vollständig verzichtet werden kann, und damit u.a. die Wasserbeständigkeit der Kontaktflächen zwischen den erhaltenen Formkörpern und den Substraten und die Haftung auf den Substraten stark verbessert wird. Durch den Verzicht auf organische Emulgatoren sind die aus den Pickering-Emulsionen (E) herstellbaren Partikel P frei von organischen Emulgatoren. Dies ist ein großer Vorteil für kosmetische Anwendungen, wo solche Verunreinigungen die Produktqualität verringern oder sogar unerwünscht sind.

Das erfindungsgemäße Verfahren zur Herstellung der Pickering-Emulsionen (E) bietet den Vorteil, dass feinteilige Emulsionen erzeugt werden können, ohne einen sehr großen Überschuss an stabilisierenden Partikeln einsetzten zu müssen. Dadurch werden nicht reaktive Verunreinigungen in den Pickering-Emulsionen (E) vermieden, die in die Umwelt gelangen können und die Wirksamkeit der vernetzbaren Emulsionen beeinträchtigen.

Die Pickering-Emulsionen (E) haben den Vorteil, dass sich deren Rheologie im Gegensatz zu bisher bekannten Systemen in Bereichen einstellen lässt, wie sie von den nichtwässrigen Systemen her bekannt sind.

Die Pickering-Emulsionen (E) haben ferner den Vorteil, dass sich die mechanischen Eigenschaften der ausgehärteten Produkte in Bereichen bewegen, wie sie von den nichtwässrigen Systemen her bekannt sind.

Die Pickering-Emulsionen (E) haben weiterhin den Vorteil, dass sie so formuliert werden können, dass keinerlei flüchtige organische Verbindungen an die Atmosphäre beim Aushärten abgegeben werden.

Die Pickering-Emulsionen (E) haben weiterhin den Vorteil, dass sie auf vielen Substraten, wie beispielsweise Papier, Textilien, mineralischen Baustoffen, wie Faserzement, Kunststoffen, Holz und vielen anderen Unterlagen, festhaftende Überzüge bilden. Dadurch eignen sie sich auch zum Verkleben vieler Substrate. Das Beschichten kann dabei z.B. durch Streichen, Walzen, Tauchen oder Spritzen erfolgen.

Die Pickering-Emulsionen (E) vernetzbarer Organopolysiloxane haben gegenüber den nicht-erfindungsgemäßen grobteiligen Emulsionen den Vorteil, dass das reaktive Organopolysiloxan in Beschichtungs-, Imprägnierungs- und Hydrophobierungsverfahren aufgrund der kleineren Tröpfchengröße tiefer in den zu behandelnden Stoff eindringen kann, wodurch die Wirksamkeit und Dauerhaftigkeit der Oberflächenbehandlung verbessert werden. Ein sehr hoher Überschuss an partikulärem Emulgator, wie im Stand der Technik verwendet, würde die Poren des zu behandelnden Stoffs verstopfen und sich auf der Oberfläche abscheiden. Dadurch wird die Wirksamkeit der Anwendung reduziert. Das erfindungsgemäße Verfahren zur Herstellung der Pickering-Emulsionen (E) ermöglicht die Herstellung von feinteiligen Emulsionen ohne Verwendung eines großen Überschusses an partikulärem Emulgator. Bei Verwendung von organischen Emulgatoren, wie im Stand der Technik verwendet, würden sich diese an der Oberfläche abscheiden. Dadurch ergeben sich unerwünschte Effekte, beispielsweise verschlechtern organische Emulgatoren die Bedruckbarkeit der Oberflächen und die Haftung beim Kleben.

Gegenstand der vorliegenden Erfindung ist auch die nach dem vorstehenden Verfahren herstellbare wässrige partikelstabilisierte Pickering Emulsion (E).

Die Tröpfchen, welche das Material (S) und den partikulären Feststoff (F) umfassen, bilden die diskontinuierliche Phase der Pickering Emulsion (E).

Die diskontinuierliche Phase enthält vorzugsweise polyadditionsfähiges, polykondensationsfähiges oder polymerisierbares Material, das zumindest ein oder mehrere Siloxane oder Silane enthält.

Vorzugsweise ist das Material (S) zumindest ein polyadditionsfähiges, polykondensationsfähiges oder polymerisierbares Siloxan der allgemeinen Formel (IV)

$$[A^1_m R^9_p SiO_{(4-p-m)/2}] \qquad (IV),$$

wobei

$A^1$ einen Wasserstoff- oder Kohlenwasserstoffrest bedeutet, der bis zu 30 C-Atome enthält und zusätzlich Heteroatome ausgewählt aus O-, S, Si, Cl, F, Br, P oder N-Atomen enthalten kann, so dass $A^1$ auch eine funktionelle Gruppe bedeuten kann, die selbst unsubstituiert oder substituiert ist,

$R^9$ Alkoxy- oder Aryloxyreste mit bis zu 18 C-Atomen, Hydroxyreste oder H bedeutet, oder die unabhängig von $A^1$ dessen Bedeutung haben kann,

$m$ und $p$ jeweils die Werte 0, 1, 2 oder 3 bedeuten

oder zumindest ein polyadditionsfähiges, polykondensationsfähiges oder polymerisierbares Silan der allgemeinen Formel (V),

$$(R^{10})_{4-n}\text{-Si-}(OR^{11})_o \qquad (V)$$

wobei **o** eine Zahl im Wert von 1, 2, 3 oder 4 bedeutet,

$R^{10}$ lineare oder verzweigte Alkylreste mit 1 bis 16 Kohlenstoffatomen, wobei nicht benachbarte Kohlenstoffatome durch Sauerstoffatome ersetzt sein können oder Arylreste bedeutet oder ein organofunktioneller Rest ist, ausgewählt aus der Gruppe Phosphonsäuremonoesterrest, Phosphonsäurediesterrest, Phosphonsäurerest, Methacryloyloxyrest, Acryloyloxyrest, Vinylrest, Mercaptorest, Isocyanatorest, wobei der Isocyanatorest gegebenenfalls zum Schutze vor chemischen Reaktionen reaktionsblockiert sein kann, Hydroxyrest, Hydroxyalkyrest, Vinylrest, Epoxyrest, Glycidyloxyrest, Morpholinorest, Piperazinorest, einen primären, sekundären oder tertiären Aminorest mit einem oder mehreren Stickstoffatomen, wobei die Stickstoffatome durch Wasserstoff oder einwertige aromatische, aliphatische oder cycloaliphatische Kohlenwasserstoffreste substituiert sein können, Carbonsäurerest, Carbonsäureanhydridrest, Aldehydrest, Urethanrest, Harnstoffrest, wobei der Rest $R^{10}$ unmittelbar am Siliziumatom gebunden sein oder durch eine Kohlenstoffkette von 1 - 6 C-Atomen davon getrennt sein kann, und

$R^{11}$ einen einwertigen linearen oder verzweigten aliphatischen oder cycloaliphatischen Kohlenwasserstoffrest, in dem nicht benachbarte Kohlenstoffatome durch Heteroatome wie O, N, P, S, Cl, F, Br oder Si ersetzt sein können, wobei die freien Valenzen der betreffenden Heteroatome durch lineare oder verzweigte Alkylreste oder durch Wasserstoffatome abgesättigt sein können oder einen einwertigen aromatischen Kohlenwasserstoffrest oder einen Rest der Form -C(=O)-$R^{12}$ bedeutet, wobei $R^{12}$ einen einwertigen linearen oder verzweigten aliphatischen oder einen cycloaliphatischen Kohlenwasserstoffrest oder einen einwertigen aromatischen Kohlenwasserstoffrest bedeutet, wobei das ausgewählte Silan oder gegebenenfalls die ausgewählten Silane in nicht hydrolysierter Form, in hydrolysierter Form oder in hydrolysierter und teilkondensierter oder hydrolysierter und kondensierter Form oder in einem Gemisch dieser Formen vorliegen können,

oder eine Zubereitung mehrerer solcher Siloxane der allgemeinen Formel (IV) und / oder Silane der allgemeinen Formel (V).

Bevorzugt handelt es sich um ein polykondensationsfähiges oder polyadditionsfähiges Silan, Siloxan, oder Zubereitung, wobei eines polyadditionsfähiges Silan, Siloxan, oder Zubereitung besonders bevorzugt ist.

Das polyadditionsfähige, polykondensationsfähige oder polymerisierbare Silan, Siloxan, oder Zubereitung, ist bei Raumtemperatur und dem Druck der umgebenden Atmosphäre, also 1013 hPa, bevorzugt flüssig und weist vorzugsweise eine Viskosität von 1 bis 500000 mm²/s, bevorzugt von 10 bis 100000 mm²/s, besonders bevorzugt von 50 bis 50000 mm²/s, insbesondere bevorzugt von 100 bis 10000 mm²/s.

In einer bevorzugten Ausführungsform wird eine Zubereitung aus mindestens einem Vinylgruppen-haltigen Organopolysiloxan und mindestens einem Organopolysiloxan mit siliciumgebundenen Wasserstoffatomen verwendet, welche eine Viskosität von 150 mm²/s bis 8000 mm²/s bei 25 °C aufweist.

In einer insbesondere bevorzugten Ausführungsform wird eine Zubereitung aus mindestens einem Vinylgruppen-haltigen Organopolysiloxan, mindestens einem Vinylgruppen-haltigen Organopolysiloxanharz und mindestens einem Organopolysiloxan mit siliciumgebundenen Wasserstoffatomen verwendet, welche eine Viskosität von 500 mm²/s bis 5000 mm²/s bei 25 °C aufweist.

Bevorzugt handelt es sich bei dem Material (S) um ein oder mehrere Siloxane aus Wiederholungseinheiten der allgemeinen Formel (IV) und / oder Silane der allgemeinen Formel (V).

Obwohl in der allgemeinen Formel (IV) nicht angegeben, können bis zu 10 Molprozent der Diorganosiloxaneinheiten durch andere, meist jedoch nur als mehr oder weniger schwer vermeidbare Verunreinigungen vorliegende Siloxaneinheiten, wie $R_3SiO_{1/2}$-, $RSiO_{3/2}$- und $SiO_{4/2}$-Einheiten, ersetzt sein, wobei **R** die vorstehend für $A^1$ angegebene Bedeutung hat.

Bevorzugt handelt es sich bei dem erfindungsgemäß eingesetzten partikulären Feststoff (F) um bei Raumtemperatur und dem Druck der umgebenden Atmosphäre, also 1013 hPa, feste Partikel.

Der partikuläre Feststoff (F) weist bevorzugt eine Löslichkeit in Wasser bei pH 7,33 und einem Elektrolythintergrund von 0,11 Mol und einer Temperatur von 37°C von kleiner 0,1 g/l, besonders bevorzugt von kleiner als 0,05 g/l, auf, bei dem Druck der umgebenden Atmosphäre, also 1013 hPa.

Bevorzugt weist der partikuläre Feststoff (F) eine Molmasse größer 10 000 g/Mol, besonders bevorzugt eine Molmasse von 50 000 bis 50 000 000 g/Mol, insbesondere von 100 000 bis 10 000 000 g/Mol, auf, jeweils gemessen bevorzugt mittels statischer Lichtstreuung.

Bevorzugt weist der partikuläre Feststoff (F) eine spezifische BET-Oberfläche von 30 bis 500 m²/g, besonders bevorzugt 100 bis 300 m²/g, auf. Die BET-Oberfläche wird nach bekannten Verfahren gemessen, bevorzugt gemäß Deutscher Industrie Norm DIN 66131 und DIN 66132.

Bevorzugt weist der partikuläre Feststoff (F) eine Mohs'sche Härte größer als 1, besonders bevorzugt größer als 4, auf.

Bevorzugt handelt es sich bei dem eingesetzten partikulären Feststoff (F) um ein Metalloxid mit einem kovalenten Bindungs-Anteil in der Metall-Sauerstoff-Bindung, wie z.B. feste Oxide der Haupt- und Nebengruppenelemente, wie der 3. Hauptgruppe, wie Bor-, Aluminium-, Gallium- und Indiumoxid, der 4. Hauptgruppe wie Siliciumdioxid, Germaniumdioxid, und Zinnoxid sowie -dioxid, Bleioxid und -dioxid, oder ein Oxid der Nebengruppenelemente, wie Titandioxid, Zirkondioxid, Hafniumdioxid, Ceroxid oder Eisenoxid.

Bei den erfindungsgemäß eingesetzten Metalloxiden handelt es sich um Silicium(IV)oxide, wie nasschemisch hergestellte, beispielsweise gefällte Kieselsäuren oder Kieselgele, oder in Prozessen bei erhöhter Temperatur hergestellte , Siliciumdioxide, wie zum Beispiel pyrogen hergestellte Kieselsäuren, wobei pyrogene Kieselsäure besonders bevorzugt ist.

Bevorzugt handelt sich bei den pyrogenen Kieselsäuren um silanisierte pyrogene Kieselsäuren mit einer Methanolzahl von kleiner 70.

Ganz besonders bevorzugt sind teilweise mit Wasser benetzbare Metalloxide wie beschrieben in EP 1433749 A1 und DE 10349082 A1.

Dabei ist die mittlere Partikelgröße des partikulären Silicium(IV)oxid Feststoffs (F) oder gegebenenfalls Aggregate der Partikel bevorzugt kleiner als der mittlere Durchmesser $d_{50}$ der Tröpfchen ohne die feinteiligen Partikel.

Die mittlere Partikelgröße des partikulären Silicium(IV)oxid Feststoffs (F) ist kleiner als 1000 nm, bevorzugt zwischen 10 nm und 800 nm, besonders bevorzugt zwischen 50 nm und 500 nm und ganz besonders bevorzugt zwischen 75 nm und 300 nm, jeweils gemessen als mittleren hydrodynamischen Äquivalentdurchmesser mittels Photonenkorrelationsspektroskopie in 173° Rückstreuung mit eine Nanosizer ZS der Fa. Malvern. Die Methanolzahl des partikulären Feststoffs (F) ist vorzugsweise kleiner als 70, bevorzugt kleiner als 50, besonders bevorzugt kleiner als 40 und insbesondere bevorzugt kleiner als 30.

Zur Bestimmung der Methanolzahl werden definierte Mischungen von Wasser mit Methanol hergestellt, und dann die Oberflächenspannungen dieser Gemische mit bekannten Methoden bestimmt. In einem getrennten Experiment werden diese Wasser-Methanol-Mischungen mit definierten Mengen an Partikeln überschichtet und unter definierten Bedingungen geschüttelt (beispielweise schwaches Schütteln mit der Hand oder mit einem Taumelmischer für ca. 1 Minute). Bestimmt wird das Wasser-Alkohol-Gemisch, bei dem die Partikel eben noch nicht einsinken und das Wasser-Alkohol-Gemisch mit höherem Alkoholgehalt, bei dem die Partikel eben einsinken. Die Oberflächenspannung des letzteren Alkohol-Wasser-Gemisches liefert die kritische Oberflächenenergie $\gamma_{crit}$ als Maß für die Oberflächenenergie $\gamma$ der Partikel. Der Methanolgehalt in Wasser ergibt die Methanolzahl.

Der Kohlenstoffgehalt des partikulären silicium (IV)oxid Feststoffs (F) ist größer als 0 Gew.%, bevorzugt 0,1 - 4 Gew.%, besonders bevorzugt 0,25 - 3,5 Gew.% und ganz besonders bevorzugt 0,5 - 3 Gew.%, gemessen mittels Elementaranalyse an den trockenen partikulären Feststoffen.

Weiterhin können die Tröpfchen zusätzlich zu mindestens einem Siloxan oder Silan, weitere Bestandteile beispielsweise ausgewählt aus Katalysatoren, Füllstoffen, Inhibitoren, Hitzestabilisatoren, Lösungsmitteln, Weichmachern, Farbpigmenten, Sensibilisatoren, Photoinitiatoren, Haftvermittlern, Thixotropiermitteln, Leitfähigkeitsadditiven, kosmetischen Stoffen, Duftstoffen, medizinischen oder kosmetischen Wirkstoffen und Siliconharzen enthalten.

Bevorzugt sind die erfindungsgemäßen partikelstabilisierten Pickering Emulsionen (E) im Wesentlichen frei von herkömmlichen, bei Raumtemperatur und dem Druck der umgebenden Atmosphäre nicht-partikulären flüssigen und festen, organischen oberflächenaktiven Substanzen, wie nichtionischen, kationischen und anionischen Emulgatoren ("organische Emulgatoren").

Organische Emulgatoren heißt hier keine Partikel und Kolloide, sondern Moleküle und Polymere, folgend der Definition von Molekülen, Polymeren, Kolloide und Partikel wie gegeben in Dispersionen und Emulsionen, G. Lagaly, O. Schulz, R. Zindel, Steinkopff, Darmstadt 1997, ISBN 3-7985-1087-3, S.1-4.

Im Allgemeinen weisen diese organischen Emulgatoren eine Größe kleiner 1 nm, eine Molmasse < 10 000 g/Mol, einen Kohlenstoffgehalt > 50 Gew.-%, bestimmbar durch Elementaranalyse, sowie eine Mohs'sche Härte kleiner als 1 auf. Zugleich weisen die organischen Emulgatoren, von denen die erfindungsgemäßen Emulsionen im Wesentlichen frei sind, zumeist eine Löslichkeit in Wasser bei 20°C und dem Druck der umgebenden Atmosphäre, also 1013 hPa, homogen oder in Micellen-Form von größer 1 Gew.-% auf.

Die erfindungsgemäßen Emulsionen (E) können solche organischen Emulgatoren bis zu einer maximalen Konzentration von kleiner als 0,1 mal, bevorzugt kleiner als 0,01 mal, besonders bevorzugt kleiner als 0,001 mal, insbesondere kleiner als 0,0001 mal der kritischen Micellkonzentration dieser organischen Emulgatoren in der Wasserphase enthalten; dies entspricht einer Konzentration dieser organischen Emulgatoren, bezogen auf das Gesamtgewicht der erfindungsgemäßen Dispersion von kleiner 10 Gew.-%, bevorzugt kleiner 2 Gew.-%, besonders bevorzugt kleiner 1 Gew.-%, insbesondere 0 Gew.-%.

Vorzugsweise wird vor der Vermischung mit Material (S) eine Vordispersion (V) des partikulären Silicium(IV)oxid Feststoff (F) in wässriger Phase (W) hergestellt.

Die Herstellung der Vordispersion (V) kann grundsätzlich gemäß den bekannten Verfahren zur Herstellung von Partikeldispersionen erfolgen, wie das Einarbeiten mittels Rührorganen mit hoher Scherwirkung wie schnelllaufende

Rührer, schnellaufende Dissolver, Rotor-Stator-Systeme, Ultraschalldispergatoren oder Kugel- bzw. Perlmühlen.

Die Konzentration des Feststoffs (F) in der Vordispersion (V) beträgt dabei zwischen 1 und 80 Gew.%, bevorzugt zwischen 10 und 60 Gew.%, besonders bevorzugt zwischen 10 und 40 Gew.% und ganz besonders bevorzugt zwischen 12 und 30 Gew.%.

Zur Herstellung der partikelstabilisierten Pickering Emulsion (E) können sämtliche dem Fachmann bekannten Methoden zur Herstellung von Emulsionen eingesetzt werden. Es zeigte sich jedoch, dass sich besonders gut geeignete Emulsionen gemäss der folgenden Verfahren erhalten lassen:

Verfahren 1:

- Vorlegen einer hochkonzentrierten Vordispersion (V), wobei das vorgelegte Volumen so bemessen ist, dass es die Gesamtmenge an benötigen feinteiligen Feststoff (F) und nur eine Teilmenge an wässriger Phase (W) enthält.
- Langsames Zudosieren des Gesamtvolumens an Material (S) unter ständigem Homogenisieren z.B. mittels eines schnellaufenden Rührers, schnellaufenden Dissolvers oder eines Rotor-Stator-Systems.
- anschließend langsames Zudosieren des gewünschten Restvolumens an wässriger Phase (W), gegebenenfalls unter ständigem Homogenisieren z.B. mittels eines schnellaufenden Rührers, schnellaufenden Dissolvers oder eines Rotor-Stator-Systems.

Verfahren 2:

- Vorlegen des Gesamtvolumens an Material (S).
- Langsames Zudosieren einer hochkonzentrierten Vordispersion (V), unter ständigem Homogenisieren z.B. mittels eines schnellaufenden Rührers, schnellaufenden Dissolvers oder eines Rotor-Stator-Systems, wobei das zudosierte Volumen so bemessen ist, dass es die Gesamtmenge an benötigen partikulären Silicium(IV)oxid Feststoff (F) und nur eine Teilmenge an wässriger Phase (W) enthält.
- anschließend langsames Zudosieren des gewünschten Restvolumens an wässriger Phase (W) gegebenenfalls unter ständigem Homogenisieren z.B. mittels eines schnellaufenden Rührers, schnellaufenden Dissolvers oder eines Rotor-Stator-Systems

Verfahren 3:

- Vorlegen des Gesamtvolumens an Material (S).
- Langsames Zudosieren einer Vordispersion (V), unter ständigem Homogenisieren z.B. mittels eines schnellaufenden Rührers, schnellaufenden Dissolvers oder eines Rotor-Stator-Systems, wobei das zudosierte Volumen so bemessen ist, dass es die Gesamtmenge an benötigen Feststoff (F) und wässriger Phase (W) enthält.

Verfahren 4:

- Vorlegen der Vordispersion (V), wobei das vorgelegte Volumen so bemessen ist, dass es die Gesamtmenge an benötigem Feststoff (F) und wässriger Phase (W) enthält.
- Langsames Zudosieren des Gesamtvolumens an Material (S) unter ständigem Homogenisieren z.B. mittels eines schnellaufenden Rührers, schnellaufenden Dissolvers, eines Rotor-Stator-Systems oder mittels Kapillaremulgators.

Verfahren 5:

- Vorlegen des Gesamtvolumens an Material (S) und der Vordispersion (V) , wobei das vorgelegte Volumen so bemessen ist, dass es die Gesamtmenge an benötigen partikulären Feststoff (F) und wässrige Phase (W) enthält.
- Gemeinsames Homogenisieren z.B. mittels eines schnellaufenden Rührers, schnellaufenden Dissolvers oder eines Rotor-Stator-Systems.

Verfahren 6:

- Vorlegen des Gesamtvolumens an Material (S) und einer hochkonzentrierten Vordispersion (V), wobei das vorgelegte Volumen so bemessen ist, dass es die Gesamtmenge an Feststoff (F) und wässrige Phase (W) enthält.
- Gemeinsames Homogenisieren z.B. mittels eines schnellaufenden Rührers, schnellaufenden Dissolvers oder eines Rotor-Stator-Systems.

- Anschließend langsames Zudosieren des gewünschten Restvolumens an wässrige Phase (W), gegebenenfalls unter ständigem Homogenisieren z.B. mittels eines schnellaufenden Rührers, schnellaufenden Dissolvers oder eines Rotor-Stator-Systems.

Bevorzugt sind Verfahren 1, 4, 5 und 6, wobei Verfahren 4 und 5 besonders bevorzugt sind und Verfahren 5 insbesondere bevorzugt ist.

Das Homogenisieren erfolgt vorzugsweise in mindestens einem Verfahrensschritt für mindestens 30 Sekunden, bevorzugt mindestens 1 Minute.

In einem optionalen Verfahrensschritt wird die erfindungsgemäße wässrige partikelstabilisierte Pickering Emulsion (E) mit wässriger Phase (W) gegebenenfalls unter ständigem Homogenisieren z.B. mittels eines schnellaufenden Rührers, schnellaufenden Dissolvers oder eines Rotor-Stator-Systems verdünnt.

Die beschriebenen Verfahren können sowohl in kontinuierlicher als auch in diskontinuierlicher Form durchgeführt werden. Bevorzugt ist die kontinuierliche Form.

Die Temperatur während des Emulgierprozesses liegt zwischen 0 °C und 80 °C, bevorzugt zwischen 10 °C und 50 °C, besonders bevorzugt zwischen 20 °C und 40 °C.

Der Emulgierprozess kann bei Normaldruck, also bei 1013 hPa, bei erhöhtem Druck oder im Vakuum durchgeführt werden. Bevorzugt ist der Prozess bei Normaldruck.

Für die Emulsion (E) werden der partikuläre Silicium(IV)oxid Feststoff (F), das Material (S) und die wässrige Phase (W) bevorzugt in den folgenden Anteilen vermischt:

(F): vorzugsweise 2 - 15 Gew.-%, bevorzugt 3 - 13 Gew.-%, besonders bevorzugt 4 - 12 Gew.-%
(S): vorzugsweise 50 - 70 Gew.-%, bevorzugt 53 - 68 Gew.-%, besonders bevorzugt 55 - 65 Gew.-%
(W): vorzugsweise 23 - 45 Gew.-%, bevorzugt 25 - 40 Gew.-%, besonders bevorzugt 27 - 36 Gew.-%

Bevorzugt ist Q1 eine beliebige Zahl von 5 bis 22, besonders bevorzugt von 8 bis 18, insbesondere von 10 bis 16.

Bevorzugt ist Q2 eine beliebige Zahl von 55 bis 70, besonders bevorzugt von 60 bis 70.

Bevorzugt ist Q3 eine beliebe Zahl von 77 bis 89, besonders bevorzugt von 82 bis 88, insbesondere von 84 bis 87.

Die Emulsion E kann nach dem Vermischen mit einer beliebigen Menge an Wasser verdünnt werden.

Das Vermischen im Verfahren wird vorzugsweise kürzer als 120 h, bevorzugt zwischen 0 h bis 48 h, besonders bevorzugt 0,1 h bis 24 h und in einer speziellen Ausführung 0,25 h bis 12 h durchgeführt.

Gegebenenfalls können im Verfahren zusätzlich zur wässrigen Phase (W), Material (S), und Feststoff (F) die Vernetzung beschleunigende und vervollständigende Katalysatoren wie oben genannt zugesetzt werden. Die Zugabe kann dabei vor der Herstellung der Pickering-Emulsion (E) direkt in das Material (S) oder die wässrige Phase (W), während der Vermischung oder nachträglich in die fertige Pickering-Emulsion erfolgen.

Die Einsatzmenge der gegebenenfalls zugesetzten Katalysatoren liegt dabei in dem für Katalysatoren typischen Mengenbereich.

Die Reaktionstemperatur während der Vermischung liegt zwischen 0 °C und 150 °C, bevorzugt zwischen 10 °C und 80 °C und besonders bevorzugt zwischen 15 °C und 60 °C.

Gegebenenfalls kann das Verfahren unter einer InertgasAtmosphäre wie Stickstoff, Argon oder Kohlendioxid durchgeführt werden. Der Sauerstoffanteil ist dann kleiner 15 Vol.%, bevorzugt kleiner 10 Vol.% und besonders bevorzugt kleiner 5 Vol.%.

Der pH-Wert der Emulsion (E) liegt zwischen pH 10 und 1, bevorzugt zwischen pH 9 und 2, besonders bevorzugt zwischen pH 7 und 2 und in einer speziellen Ausführung zwischen pH 6 und 2,5.

Gegebenenfalls können der Emulsion (E) wasserlösliche organische Lösungsmittel wie Alkohole wie Methanol, Ethanol oder i-Propanol oder Ketone wie Aceton oder MEK oder Ether wie THF oder andere zugesetzt werden. Diese können entweder unmittelbar nach Abschluss der Herstellung der Emulsion (E), oder während der Vermischung zugesetzt werden.

Gegebenenfalls können der Emulsion (E) Dispergierhilfsmittel, Schutzkolloide oder ähnliches zugesetzt werden. Diese können entweder unmittelbar nach Abschluss der Herstellung der Emulsion (E), oder während der Vermischung zugesetzt werden.

Bevorzugt enthält die Emulsion (E) weniger als 5 Gew.% Dispergierhilfsmittel, Schutzkolloide, oder ähnliches, besonders bevorzugt enthält die Dispersion der erfindungsgemäßen Partikel weniger als 1 Gew.% Dispergierhilfsmittel, Schutzkolloide, oder ähnliches, ganz besonders bevorzugt enthält die Dispersion der erfindungsgemäßen Partikel weniger als 0,1 Gew.% Dispergierhilfsmittel, Schutzkolloide, oder ähnliches und in einer speziellen Ausführung ist die Emulsion (E), frei von Dispergierhilfsmitteln, Schutzkolloiden, oder ähnlichem.

Gegebenenfalls können der Emulsion (E) anorganische oder organische Elektrolyte zugesetzt werden. Diese können entweder unmittelbar nach Abschluss der Herstellung der Pickering-Emulsion, während der Reaktionsphase oder nach Abschluss der Reaktionsphase zugesetzt werden. Die Ionenstärke der Dispersion liegt dabei zwischen 0,01 mmol/l und 1 mol/l, bevorzugt zwischen 0,1 mmol/l und 500 mmol/l und besonders bevorzugt zwischen 0,5 mmol/l und 100 mmol/l.

Gegebenenfalls können der Emulsion (E) Verdickungsmittel zugesetzt werden. Dabei kann es sich um anorganische oder organische Verdickungsmittel handeln, die bei Raumtemperatur und dem Druck der umgebenden Atmosphäre, also bei 1013 hPa, in fester oder flüssiger Form vorliegen. Diese können entweder unmittelbar nach Abschluss der Herstellung der Emulsion (E), oder während der Vermischung zugesetzt werden.

Gegebenenfalls können dem Bestandteil (S) vor dem Emulgieren der Emulsion (E) Füllstoffe zugesetzt werden, es handelt sich um Mengen von vorzugsweise 0,1 bis 200 Gewichtsteilen, besonders bevorzugt 0,5 bis 100 Gewichtsteilen, jeweils bezogen auf 100 Gewichtsteile Material (S). Die eingesetzte Menge an Füllstoff kann in weiten Bereichen variiert werden und richtet sich insbesondere nach dem jeweiligen Anwendungszweck der erfindungsgemäßen Emulsion (E).

Die Zugabe der Füllstoffe und Zusatzstoffe kann vor der Herstellung der Pickering-Emulsion (E) direkt in das Material (S) oder in die wässrige Phase (W), während der Emulgierung oder nachträglich in die fertige Pickering-Emulsion (E) erfolgen.

Beispiele für Füllstoffe sind nichtverstärkende Füllstoffe, wie zum Beispiel Füllstoffe mit einer BET-Oberfläche von bis zu 50 m$^2$/g, wie Quarz, Diatomeenerde, Calciumsilikat, Zirkoniumsilikat, Zeolithe, Aluminiumoxid, Titanoxid, Eisenoxid, Zinkoxid, Bariumsulfat, Calciumcarbonat, Gips, Siliciumnitrid, Siliciumcarbid, Bornitrid, Glas- und Kunststoffpulver.

Beispiele für verstärkende Füllstoffe sind die vorstehend für den partikulären Feststoff (F) genannte Feststoffe mit einer spezifische BET-Oberfläche von 30 bis 500 m$^2$/g, sowie Siliconharze.

Beispiele für Siliconharze sind MQ-, MT-, T, MDQ-, MDT-, MTQ und MDTQ-Siliconharze, wobei M ausgewählt wird aus $R_3SiO_{1/2}$- und $HR_2SiO_{1/2}$- und $R^1R_2SiO_{1/2}$-, D ausgewählt wird aus $R^1RSiO_{1/2}$- und $R_2SiO_{1/2}$- und $HRSiO_{1/2}$-, T ausgewählt wird aus $RSiO_{3/2}$- und $R^1SiO_{3/2}$- und $HSiO_{3/2}$- und Q gleich $SiO_{4/2}$-Einheiten sind, wobei **R** unabhängig voneinander, gleich oder verschieden, ein von aliphatischen Kohlenstoff-Kohlenstoff-Mehrfachbindungen freien, organischen oder anorganischen Rest, und **R$^1$** unabhängig voneinander, gleich oder verschieden einen einwertigen, substituierten oder nicht substituierten, SiC-gebundenen Kohlenwasserstoffrest mit mindestens einer aliphatischen Kohlenstoff-Kohlenstoff-Mehrfachbindung bedeuten.

Bevorzugt handelt es sich bei Rest R um einen einwertigen, von aliphatischen Kohlenstoff-Kohlenstoff-Mehrfachbindungen freien, SiC-gebundenen, gegebenenfalls substituierten Kohlenwasserstoffrest mit 1 bis 18 Kohlenstoffatomen, besonders bevorzugt um einen einwertigen, von aliphatischen Kohlenstoff-Kohlenstoff-Mehrfachbindungen freien, SiC-gebundenen Kohlenwasserstoffrest mit 1 bis 6 Kohlenstoffatomen, insbesondere um den Methyl- oder Phenylrest.

Bei Rest R$^1$ kann es sich um beliebige, einer Anlagerungsreaktion (Hydrosilylierung) mit einer SiHfunktionellen Verbindung zugängliche Gruppen handeln. Bevorzugt handelt es sich bei Rest R$^1$ um Alkenyl- und Alkinylgruppen mit 2 bis 16 Kohlenstoffatomen, wie Vinyl-, Allyl-, Methallyl-, 1-Propenyl-, 5-Hexenyl-, Ethinyl-, Butadienyl-, Hexadienyl-, Cyclopentenyl-, Cyclopentadienyl-, Cyclohexenyl-, Vinylcyclohexylethyl-, Divinylcyclohexylethyl-, Norbornenyl-, Vinylphenyl- und Styrylreste, wobei Vinyl-, Allyl- und Hexenylreste besonders bevorzugt verwendet werden. Die als Füllstoffe geeigneten Siliconharze sind bei 20°C und 1013 hPa fest.

Der Anteil der erfindungsgemäßen Tröpfchen in der Emulsion (E), bestehend aus der Summe aus eingesetzten Material (S) und partikulärem Feststoff (F) liegt zwischen 0,1 Gew.% und 99 Gew.%, bevorzugt zwischen 5 Gew.% und 90 Gew.% und besonders bevorzugt zwischen 10 Gew.% und 80 Gew.%.

Gegebenenfalls kann die fertige Emulsion (E) weiterhin unter Rühren gelagert werden. Dies kann beispielsweise mittels Balken- oder Ankerrührer erfolgen.

Gegenstand der vorliegenden Erfindung ist auch ein Verfahren zur Herstellung von Partikeln (P) aus einem Kern (K) enthaltend polymeres Material und einer Hülle (H) aus partikulärem Feststoff (F),
bei dem in einem zweiten Schritt das polyadditionsfähige, polykondensationsfähige oder polymerisierbare Material (S) der wässrigen partikelstabilisierten Pickering Emulsion (E), das ausgewählt wird aus Siloxan und Silan, einer Polyaddition Polymerisation oder Kondensation unterzogen wird.

Vorzugsweise ist der zweite Schritt so auszuführen, dass der Gehalt an polymerisierbarem Material (S) in der wässrigen partikelstabilisierten Pickering Emulsion (E) kleiner als 70 Gew.-%, bevorzugt kleiner als 60 Gew.-%, besonders bevorzugt kleiner als 50 Gew.-% und insbesondere bevorzugt kleiner als 40 Gew.-% ist. Vorzugsweise wird die partikelstabilisierten Pickering Emulsion (E) vor dem zweiten Schritt mit Wasser oder einem wasserlöslichen organischen Lösungsmittel verdünnt, bevorzugt mit Wasser.

Das polymerisierbare Material (S) wird in einem zur Herstellung der Partikeln (P) geeigneten Verfahren nach dem Vermischen im ersten Schritt einer Polyaddition unterzogen polymerisiert oder kondensiert.

Gegebenenfalls müssen die Siloxane oder Silane vor der Kondensation hydrolysiert werden, wenn es sich dabei beispielsweise um alkoxy- oder acetoxysubstituierte Silane oder Siloxane handelt. Bei ausreichend reaktiven Silanen und Siloxanen, bewirkt dabei bereits das vorhandene Wasser gegebenenfalls die Hydrolyse und nachfolgend die Kondensation. Bei weniger reaktiven Silanen und Siloxanen sind Katalysatoren erforderlich, die gegebenenfalls die Hydrolyse und die Kondensation der Siloxane und Silane bewirken. Diese Katalysatoren können sowohl Säuren als auch Basen sein oder auch Metallkatalysatoren, wie Gruppe IV Übergangsmetallkatalysatoren oder Zinnkatalysatoren, wie sie üblicherweise zur Beschleunigung von Hydrolysen, Kondensationsreaktionen oder Umesterungsreaktionen verwendet werden. Als Säuren oder Basen kommen neben den bekannten Mineralsäuren und Metallsalzen auch saure oder basische Silane oder Siloxane in Frage.

Bevorzugte basische Katalysatoren sind NaOH, KOH, Ammoniak und $NEt_3$.

Bevorzugte saure Katalysatoren sind p-Toluolsulfonsäure, wässrige oder gasförmige HCl, Schwefelsäure.

Falls das Verfahren eine Polymerisationsreaktion umfasst, kann es sich dabei beispielsweise um eine radikalische Polymerisationreaktion eines olefinisch ungesättigten Siloxans oder Silans handeln.

Falls das Material (S) ein polyadditionsfähiges Siloxan oder Silan umfasst, werden dem Material (S) vorzugsweise Übergangsmetallkatalysatoren wie z.B. Platinkatalysatoren, zugesetzt, insbesondere dann, wenn es Siloxane enthält, die in einer Hydrosilylierungsreaktion miteinander reagieren können.

Das Verfahren ist so auszuführen, dass der Siloxan- oder Silanphase stabilisierende feinteilige partikuläre Feststoff (F) während der Kondensationsreaktion, der Polymerisationsreaktion oder der Polyadditionsreaktion mit der Oberfläche der die Kerne (K) bildenden Kondensations- oder Polymerisationsprodukte reagiert oder zumindest eine stabile Wechselwirkung, wie Wasserstoffbrückenbindungen, van der Waals Wechselwirkungen oder eine andere gerichtete Wechselwirkung, bzw. eine Kombination solcher gerichteten Wechselwirkungen eingeht, so dass der Feststoff (F) auf den Kernen (K) aus den Kondensationsprodukten oder Polymerisationsprodukten des Materials (S) verankert ist.

Gegebenenfalls kann die Oberfläche der Partikel (P) durch Behandlung mit reaktiven Silanen oder Siloxanen modifiziert werden. Diese können entweder unmittelbar nach Abschluss der Herstellung der Pickering-Emulsion, während der Reaktionsphase oder nach Abschluss der Reaktionsphase im zweiten Schritt zugesetzt werden, vor der Isolierung der Partikel (P) oder nach der Isolierung der Partikel in flüssiger oder fester Phase. Die Behandlung ist dabei so durchzuführen, dass eine kovalente, chemische Anbindung des Silans oder Siloxans an den Partikeln erfolgt. Entsprechende Methoden und Verfahren sind dem Fachmann bekannt.

Gegebenenfalls kann die Pickering Emulsion (E) nach Abschluss der Reaktionsphase im zweiten Schritt weiterhin unter Rühren gelagert werden. Dies kann beispielsweise mittels Balken- oder Ankerrührer erfolgen.

In einer bevorzugten Ausführungsform werden die Partikel (P) isoliert, vorzugsweise durch Sedimentation, Abfiltrieren oder Zentrifugieren, bevorzugt durch Filtration oder Zentrifugieren, besonders bevorzugt durch Zentrifugation.

Nach dem Isolieren werden die Partikel (P) vorzugsweise mit einer Waschflüssigkeit gewaschen, die vorzugsweise ausgewählt wird aus VE-Wasser, Methanol, Ethanol und Gemischen davon.

In einer bevorzugten Ausführungsform werden die Partikel (P) in Pulverform von der wässrigen Phase (W)isoliert. Dies kann beispielsweise mittels Filtration, Sedimentation, Zentrifugieren oder durch Entfernen der flüchtigen Bestandteile durch Trocknen in Öfen oder Trocknern oder durch Sprühtrocknung oder durch Anlegen eines entsprechenden Vakuums erfolgen.

Durch Sprühtrocknen lässt sich ohne weitere Bearbeitung eine sehr hohe Feinheit der Partikel (P) erreichen. Statisch getrocknete Partikel (P) neigen zur Ausbildung loser Agglomerate, die durch geeignete Mahlverfahren, zum Beispiel Kugelmühle oder Luftstrahlmühle deagglomeriert werden können.

Die Partikel (P) sind insbesondere dadurch gekennzeichnet, dass sie einen mittleren Partikeldurchmesser $d_{50}$ von 0,5 bis 9 $\mu$m, vorzugsweise von 0,8 bis 8,5 $\mu$m bevorzugt 1,0 bis 8,0 $\mu$m, besonders bevorzugt von 1,5 bis 7 $\mu$m und insbesondere bevorzugt von 2 bis 6 $\mu$m aufweisen, gemessen mit einem Camsizer X2 von Retsch Technology (Messprinzip: Dynamische Bildanalyse nach ISO 13322-2, Messbereich: 0,8 $\mu$m - 30 mm, Art der Analyse: Trockenmessung von Pulvern und Granulaten, Dispergierdruck = 2 bar) .

Vorzugsweise weisen die Partikel (P) ein schmale Partikelgrößenverteilungsbreite auf, welche dadurch gekennzeichnet ist, dass die Verteilungsbreite ($d_{90}$-$d_{10}$) kleiner als 20 $\mu$m, bevorzugt kleiner als 17 $\mu$m, besonders bevorzugt kleiner als 15 $\mu$m, und insbesondere bevorzugt kleiner als 8 $\mu$m ist.

Die Partikel (P) sind insbesondere dadurch gekennzeichnet, dass die eingesetzten partikulären Feststoffe (F) im wesentlich an der Oberfläche der Polymer-Partikel (P) gebunden sind. Die Verteilung der eingesetzten partikulären Feststoffe (F) lässt sich aus TEM-Aufnahmen von Dünnschliffen von eingebetteten erfindungsgemäßen Partikeln erhalten. Der partikuläre Feststoff (F) taucht vorzugsweise mehr als 10 nm, bevorzugt mehr als 20 nm, besonders bevorzugt mehr als 30 nm in das vernetzte Polymerisationsprodukt ein und ragt vorzugsweise mehr als 10 nm, bevorzugt mehr als 20 nm, besonders bevorzugt mehr als 30 nm aus dem vernetzten Polymerisationsprodukt heraus, jeweils gemessen von der äußeren Grenze des vernetzten Polymerisationsprodukts, und ist dadurch an der Oberfläche des Partikels (P) fest gebunden. Dies ist ein wesentlicher Vorteil gegenüber einem kommerziell erhältlichen Produkt, bei dem der fertige Siliconelastomer Partikel mit einer Kieselsäure nachbehandelt wird. Bei diesem Produkt taucht die Kieselsäure nicht in das Siliconelastomer ein und ist dadurch nicht fest an der Oberfläche gebunden.

Die Partikel (P) sind im Wesentlichen sphärisch. Vorzugsweise ist die Sphärizität SPHT3 mindestens 0,8, bevorzugt mindestens 0,82, bestimmbar gemäß ISO 9276-6 mit einem Camsizer X2 von Retsch Technology.

Die Partikel (P) weisen im Vergleich zu nicht-erfinderischen Silica-beschichteten Partikel nach dem Stand der Technik, die mit gleicher Menge an partikulärem Feststoff (F) hergestellt wurden, eine deutlich geringere Partikelgröße auf. Dadurch können diese Partikel für Anwendungen verwendet werden, für die vergleichsweise großen Silica-beschichteten Partikel nach dem Stand der Technik nicht geeignet sind. Die erfinderischen Partikel können beispielsweise bei der Verarbeitung oder beim Aufbringen deutlich kleinere Öffnungen, Spalte oder Düsen passieren und ermöglichen dadurch die Erzeugung deutlich feinerer Strukturen oder Oberflächenbeschichtungen, als dies mit den vergleichsweise großen Silica-beschichteten Partikel nach dem Stand der Technik möglich ist.

Die erfindungsgemäßen Partikel (P) sind amphiphil, das heißt, sie sind sowohl hydrophil (also wasserliebend) als auch lipophil (also fettliebend). Dies bedeutet, dass die erfindungsgemäßen Partikel sowohl in polaren Lösungsmitteln, wie zum Beispiel Wasser oder Alkohole, als auch in unpolaren Lösungsmitteln, wie zum Beispiel aliphatische Kohlenwasserstoffe, oder Polydimethylsiloxanöle, ohne Zusatz weiterer Dispergierhilfsmittel oder Additive, wie zum Beispiel organische Emulgatoren oder andere oberflächenaktive Substanzen, gut dispergierbar sind. Dies ist ein wesentlicher Vorteil der erfindungsgemäßen Partikel (P) im Vergleich zu unbeschichteten Siliconelastomer Partikeln, welche sehr hydrophob sind und ohne Verwendung unerwünschter Hilfsstoffe, wie organische Emulgatoren, nicht in polaren Lösungsmitteln, wie zum Beispiel Wasser oder Alkohole, dispergierbar sind. Bei den erfindungsgemäßen Partikeln (P) ist der partikuläre Feststoff (F) an der Oberfläche fest gebunden, dadurch bleiben die amphiphilen Eigenschaften bei der Dispergierung in einem Lösungsmittel erhalten. Dies ist ein wesentlicher Vorteil gegenüber Siliconelastomer Partikel nach dem Stand der Technik, die nach der Härtung mit Silica nachbehandelt werden, da bei diesen nicht erfindungsgemäßen Partikel die absorbierte Silica nicht permanent gebunden ist. Nachteilig an diesen Partikeln nach dem Stand der Technik ist, dass nicht-permanent gebundene Silica bei der Dispergierung der Partikel in einem Lösungsmittel ganz oder teilweise abgelöst wird, wodurch die Partikel stark hydrophob werden und nicht mehr in polaren Lösungsmitteln, wie zum Beispiel Wasser, dispergierbar sind. Gegenstand der vorliegenden Erfindung sind auch additionsvernetzt e , kondensationsvernetzte oder polymerisationsvernetzte Siliconharzpartikel (P1) mit einem mittleren Durchmesser d50 von höchstens 9 μm, herstellbar nach dem vorstehend beschriebenen Verfahren. Insbesondere bevorzugt sind kondensationsvernetzte Siliconharzpartikel (P1).

Das polyadditionsfähige, polykondensationsfähige oder polymerisierbare Material (S) ist dabei zu einem duroplastischen Siliconharz vernetzt.

Bevorzugte Beispiele für Siliconharze, welche sich für die Siliconharzpartikel (P1) eignen, sind die vorstehend bei den verstärkenden Füllstoffen genannten Siliconharze, welche kondensationsvernetzt sind, insbesondere die reinen T-Harze.

Die wässrige partikelstabilisierte Pickering Emulsion (E) kann für alle Zwecke eingesetzt werden, für die auch bisher wässrige Dispersionen verwendet werden. Die wässrige partikelstabilisierte Pickering Emulsion (E) kann eingesetzt werden für kosmetische und pharmazeutische Anwendungen, Putzund Reinigungsmittel oder Anwendungen zur Veränderung der Grenzflächeneigenschaften von festen und flüssigen Substraten, wie z.B. Hydrophobiermittel, Haftvermittlern, Trennmitteln, Papierbeschichtungen oder Schaumkontrollmitteln, zur Herstellung von w/o/w- bzw. o/w/o-Mehrfachemulsionen, beispielsweise als Controll-Release-Systeme oder zur Segregation von Reaktivstoffen.

Die Pickering Emulsion (E) vernetzt nach Entfernen von Wasser und härtet zu Elastomeren oder Harzen aus. So kann die wässrige partikelstabilisierte Pickering Emulsion (E) beispielsweise als Dichtungs- und Klebmasse, Farbe, Anstrichsystem und als elektrisch isolierendes bzw. leitende, hydrophobe, klebrige Stoffe abweisendes Beschichtungssystem oder als Grundlage bzw. Zusatz zu solchen Systemen dienen. Auch können Formkörper hergestellt werden durch Vernetzung der Emulsion (E).

Die Pickering Emulsion (E) und die Partikel (P) werden insbesondere in kosmetischen Produkten verwendet.

Die Partikel (P) zeigen ein sehr vorteilhaftes Verhalten, insbesondere für kosmetische Anwendungen. Sie neigen nicht zur Agglomeration oder zum verblocken und sind dadurch außerordentlich leicht zu verteilen und bewirken ein samtiges Hautgefühl. Dieses Verhalten ist bei nicht-erfindungsgemäßen unbeschichteten Siliconelastomer Partikeln nicht zu beobachten. Diese ballen beim Verteilen auf der Haut zusammen und bewirken ein ungünstiges Gefühl.

Im Vergleich zu nicht-erfinderischen Partikeln, die nicht mit Silica beschichtet sind, weisen die Partikel (P) eine größere Oberfläche auf. Dadurch können die Partikel (P) eine größere Menge an Hautflüssigkeiten absorbieren und bewirken ein trockenes Hautgefühl über längere Zeit.

Im Vergleich zu nicht-erfinderischen Partikeln, die nicht mit Silica beschichtet sind, können Silica-beschichtet Partikel eine größere Menge funktioneller Stoffe auf der Silica-Oberfläche absorbieren, beispielsweise Duftstoffe, Pflegestoffe, Vitamine oder UV-Absorber, oder medizinische Wirkstoffe. Diese Stoffe können dann auf der Haut freigesetzt werden.

Im Vergleich zu nicht-erfinderischen Partikeln, die nicht mit Silica beschichtet sind, verhalten sich die Silica-beschichtet Partikel amphiphil, d.h. sie sind sowohl in öligen, als auch in wässrigen Flüssigkeiten gut dispergierbar.

Im Vergleich zu nicht-erfinderischen Partikeln, die nicht mit Silica beschichtet sind, ist die Oberfläche der Silica-beschichtet Partikel besser durch Flüssigkeiten benetzbar. Dadurch können sie wesentlich einfacher und schneller in Flüssigkeiten, wie beispielsweise kosmetische Formulierungen, dispergiert werden und sie absorbieren auch deutlich schneller und einfacher Flüssigkeiten auf der Oberfläche, beispielsweise absorbieren sie Sebum bei kosmetischer Applikation auf der Haut.

Die Partikel (P) weisen im Vergleich zu nicht-erfinderischen Silica-beschichteten Partikel nach dem Stand der Technik eine deutlich geringere Partikelgröße auf. Dadurch besitzen sie in kosmetischen Anwendungen beim Verteilen auf der Haut ein angenehmeres und weicheres Hautgefühl und ergeben ein gleichmäßigeres Hautbild durch die feinere Abdeckung der Haut. Unebenheiten und Flecken auf der Haut werden dadurch besser abgedeckt.

Die Partikel (P) besitzen eine größere Oberfläche im Vergleich zu nicht-erfinderischen Silica-beschichteten Partikel nach dem Stand der Technik und können dadurch größere Mengen Hautflüssigkeiten oder funktionelle Stoffe oder medizinische Wirkstoffe aufnehmen.

**Beispiele**

Feststoffgehalt:

10 g wässrige Dispersion werden in einer Porzellanschale mit der gleichen Menge Ethanol versetzt und in einem $N_2$-gespülten Trockenschrank bei 150 °C zur Gewichtskonstanz eingedampft. Die Masse $m_s$ des trockenen Rückstandes ergibt den Feststoffgehalt gemäß Feststoffgehalt / % = $m_s$ * 100 / 10 g.

mittl. Partikeldurchmesser ($d_{50}$-Wert):

Die Bestimmung des $d_{50}$-Werts erfolgte mit einem Camsizer X2 von Retsch Technology (Messprinzip: Dynamische Bildanalyse nach ISO 13322-2, Messbereich: 0,8 $\mu$m - 30 mm, Art der Analyse:
Trockenmessung von Pulvern und Granulaten, Dispergierdruck = 2 bar) .

Methanolzahl:

Zur Bestimmung der Methanolzahl werden definierte Mischungen von Wasser mit Methanol hergestellt. In einem getrennten Experiment werden diese Wasser-Methanol-Mischungen mit definierten Mengen an getrockneten Partikeln überschichtet und unter definierten Bedingungen geschüttelt (beispielweise schwaches Schütteln mit der Hand oder mit einem Taumelmischer für ca. 1 Minute). Bestimmt werden das Wasser-Alkohol-Gemisch, bei dem die Partikel eben noch nicht einsinken und das Wasser-Alkohol-Gemisch mit höherem Alkoholgehalt, bei dem die Partikel eben einsinken. Letzterer Methanolgehalt in Wasser ergibt die Methanolzahl.

**Beispiel 1:** Herstellung einer wässrigen Kieselsäuredispersion

1300 g einer teilhydrophoben pyrogenen Kieselsäure mit einem Restsilanolgehalt von 71% und einem Kohlenstoffgehalt von 0,95%, erhalten durch Umsetzung einer hydrophilen Ausgangskieselsäure mit einer spezifischen BET-Oberfläche von 200 $m^2$/g (erhältlich unter der Bezeichnung HDK® N20 bei der Wacker-Chemie GmbH, München) mit Dimethyldichlorsilan gemäß EP 1433749 A1, werden portionsweise an einem Dissolver bei 650 rpm in 5200 g vollentsalztes (VE-)Wasser eingerührt. Nach vollständiger Zugabe der Kieselsäure wird noch 60 min bei 650 rpm nachdispergiert. Es wird eine hochviskose Dispersion mit 20% Feststoffgehalt und einem pH Wert von 4,2 erhalten.

**Beispiel 2:** Allgemeine Vorgehensweise zur Herstellung einer Pickering Emulsion von polymerisationsfähigen, polykondensationsfähigen oder polyadditionsfähigen Silanen und/oder Siloxanen mittels Ultra-Turrax im Batch-Verfahren

Schritt 1: Die in Beispiel 1 beschriebenen

Kieselsäuredispersion wird in einem geeigneten 1000 mL Edelstahlgefäß eingewogen und mit einem Ultra-Turrax T50 bei 10000 U/min für 10 min aufgerührt. Dabei nimmt die Viskosität der Dispersion ab. Optional wird VE-Wasser zugegeben und homogen vermischt. Die vermischte Siliconölkomponente hergestellt nach einem der Beispiele 7, 8 oder 9 wird zur aufgerührten Kieselsäuredispersion gegeben und anschließend mittels Ultra-Turrax 10 min bei 10000 U/min und Eiskühlung homogenisiert. Dabei sollte die Temperatur der Mischung nicht über 35 °C steigen. Es resultiert eine weiße, hochviskose Masse (Emulsion (E)).

Schritt 2: Die hochviskose Masse aus Schritt 1 wird durch Zugabe von drei gleich großen Portionen VE-Wasser auf 30% Siliconöl-Gehalt verdünnt. Nach jeder Portion VE-Wasser wird 3 Minuten bei 6000 U/min gerührt. Es entsteht eine dünnflüssige, weiße O/W-Emulsion.

**Beispiel 3:** Allgemeine Vorgehensweise zur Herstellung einer Pickering Emulsion von polymerisationsfähigen, polykondensationsfähigen oder polyadditionsfähigen Silanen und/oder Siloxanen mittels Dissolver

Schritt 1: Die in Beispiel 1 beschriebenen

Kieselsäuredispersion wird in ein geeignetes Rührgefäß eingewogen und mit einem Labo-Top Planetendissolver der Fa. PC Laborsystem, CH, bei 6000 U/min für 10 min aufgerührt. Dabei nimmt die Viskosität der Dispersion ab. Optional wird VE-Wasser zugegeben und homogen vermischt. Die vermischte Siliconölkomponente hergestellt nach einem der Beispiele 7, 8 oder 9 wird zur aufgerührten Kieselsäuredispersion gegeben und am Dissolver 10 min bei 6000 U/min und Wasserkühlung homogenisiert. Dabei sollte die Temperatur der Mischung nicht über 35 °C steigen. Es resultiert eine weiße, hochviskose Masse.

Schritt 2: Die hochviskose Masse aus Verfahrensschritt 1 wird bei 1000 U/min durch Zugabe von drei gleich großen Portionen VE-Wasser auf 30% Siliconöl-Gehalt verdünnt. Nach jeder Portion VE-Wasser wird 3 Minuten bei 1000 U/min gerührt. Es entsteht eine dünnflüssige, weiße O/W-Emulsion.

**Beispiel 4:** Allgemeine Vorgehensweise zur Herstellung einer Pickering Emulsion von polymerisationsfähigen, polykondensationsfähigen oder polyadditionsfähigen Silanen und/oder Siloxanen mittels Ultra-Turrax im Dosier-Verfahren

Im Unterschied zu Beispiel 2 werden die vermischten Ölkomponenten über 15 min langsam unter gleichzeitiger Homogenisierung mit einem Ultra-Turrax bei 10000 U/min zudosiert.

**Beispiel 5:** Allgemeine Vorgehensweise zur Herstellung von Silica-beschichteten Siliconpartikeln aus Pickering Emulsionen von polykondensationsfähigen Siliconölkomponenten

Aus einer polykondensationsfähigen Siliconölkomponente hergestellt nach Beispiel B8 oder B9 wird gemäß Beispiel B2 oder Beispiel B3 eine polykondensationsfähige Pickering Emulsion hergestellt. Zu 250 g dieser polykondensationsfähigen Pickering Emulsion werden 1,5 g p-Toluolsulfonsäure gegeben. Die Reaktionsmischung wird 24 h bei Raumtemperatur gerührt. Es resultiert eine weiße, dünnflüssige Dispersion. Die Partikel werden abfiltriert und 24 h bei 80 °C im Trockenschrank getrocknet. Man erhält ein feines, weißes Pulver.

**Beispiel 6:** Allgemeine Vorgehensweise zur Herstellung von Silica-beschichteten Siliconpartikeln aus Pickering Emulsionen von polyadditionsfähigen Silanen und/oder Siloxanen

Aus der polyadditionsfähigen Siliconölkomponente B7 wird gemäß Beispiel B2 oder Beispiel B3 eine polyadditionsfähige Pickering Emulsion hergestellt. 250 g dieser polyadditionsfähigen Pickering Emulsion werden 24 h bei 80 °C gerührt. Es resultiert eine weiße, dünnflüssige Dispersion. Die Partikel werden abfiltriert und 24 h bei 80 °C im Trockenschrank getrocknet. Man erhält ein feines, weißes Pulver.

**Beispiel 7:** Herstellung der polyadditionsfähigen Siliconölkomponente B7

An einem Laborrührwerk wurden 375 g eines vinyldimethylsiloxyterminierten Polydimethylsiloxans mit einer Viskosität von 1 000 mPas (25°C) und 264 g eines vinyldimethylsiloxyterminierten Polydimethylsiloxans mit einer Viskosität von 20 000 mPas (25°C) homogen vermischt. Anschließend wurden 274 g eines Vinylgruppen-haltigen Silicon-harzes der Zusammensetzung $[Me_3SiO_{1/2}]_{26,65}$ $[ViMe_2SiO_{1/2}]_{3,72}$ $[SiO_{4/2}]_{42,78}$ $[HO_{1/2}]_{1,02}$ $[EtO_{1/2}]_{5,93}$ (Molekulargewicht laut SEC (Eluent Toluol): Mw = 5300 g/mol; Mn = 2560 g/mol) zugegeben und bis zur vollständigen Lösung gerührt. Danach wurden 0,45 g eines Platin-sym-Divinyltetramethyldisiloxan-Komplexes enthaltenden Lösung, die 1 Gew.-% Pt enthält, und 5,6 g 1,1,3,3-Tetramethyl-1,3-divinyldisiloxan zugegeben und homogen verrührt.

Die so erhaltene Grundmasse wurde mit 114 g eines Mischpolymerisats aus Dimethylsiloxy-, Methylhydrogensiloxyund Trimethylsiloxy-Einheiten mit einer Viskosität von 40 mPas bei 25°C und einem SiH-Gehalt von 0,40% homogen vermischt. Die entstandene reaktive Mischung ist nicht lagerfähig und wurde innerhalb einer Stunde nach Herstellung gemäß Beispiel 2 oder Beispiel 3 emulgiert.

**Beispiel 8:** Herstellung der polykondensationsfähigen Siliconölkomponente B8

Als polykondensationsfähige Siliconmasse B8 wurde ein methoxygruppenhaltiges oligomeres Kondensationsprodukt aus Methyltrimethoxysilan mit einem Molekulargewicht von Mw = 1200 und einem Methoxygruppengehalt von ca. 30 Gewichtsprozent verwendet, welches nach den üblichen Methoden hergestellt wurde.

**Beispiel 9:** Herstellung der polykondensationsfähigen Siliconölkomponente B9

Als polykondensationsfähige Siliconmasse B9 wurde ein Siliconharz der Zusammensetzung $[MeSiO_{3/2}]_{23}$ $[EtO_{1/2}]_{27}$ (Molekulargewicht laut SEC (Eluent Toluol): Mw = 2560 g/mol; Mn = 900 g/mol; Viskosität (dynamisch, 25 °C) 25 mPa.s) verwendet, welches nach den üblichen Methoden hergestellt wurde.

Tabelle 1a (erfindungsgemäße Beispiele)

| Beispiel | 10 | 11 | 12 | 13 | 14 | 15 |
|---|---|---|---|---|---|---|
| Verwendete Siliconölkomponente | B7 | B7 | B8 | B7 | B7 | B7 |
| Viskosität Siliconölkomponente (mPa*s) | 3500 | 3500 | 30 | 3500 | 3500 | 3500 |
| Vernetzungstyp * | a | a | k | a | a | a |
| Mischaggregat | Dissol -ver | Ultra - Turra x | Ultra - Turra x | Dissol -ver | Ultra - Turra x | Dissol -ver |
| Drehzahl | 6000 | 10000 | 10000 | 6000 | 10000 | 6000 |
| Menge Siliconölkomponente in Schritt 1 (g) | 320 | 188 | 188 | 320 | 170 | 319 |
| Menge Kieselsäuredispersion aus Beispiel 1 in Schritt 1(g) | 190 | 112 | 112 | 240 | 130 | 226 |
| Menge Wasser in Schritt 1 (g) | - | - | - | - | - | - |
| Herstellung Pickering Emulsion gemäß Beispiel | 3 | 2 | 2 | 3 | 2 | 3 |
| Herstellung Partikel gemäß Beispiel | 6 | 6 | 5 | 6 | 6 | 6 |
| Q1 | 11,9 | 11,9 | 11,9 | 15,3 | 15,3 | 17,0 |
| Q2 | 67,8 | 67,8 | 67,8 | 62,5 | 62,0 | 62,5 |
| Q3 | 86,4 | 86,4 | 86,4 | 86,4 | 85,9 | 89,0 |
| d50 / $\mu$m | 3, 3 | 4,5 | 3,1 | 3, 4 | 3,5 | 3, 8 |
| Verteilungsbreite (d90-d10) | 3, 9 | 5,6 | 4,5 | 3, 8 | 3,7 | 5, 5 |
| * Vernetzungstyp: a = additionsvernetzend; k = kondensationsvernetzend | | | | | | |

Tabelle 1b (erfindungsgemäße Beispiele)

| Beispiel | 16 | 17 | 18 | 19 | 20 |
|---|---|---|---|---|---|
| Verwendete Siliconölkomponente | B7 | B7 | B8 | B9 | B8 |
| Viskosität Siliconölkomponente (mPa*s) | 3500 | 3500 | 30 | 30 | 30 |
| Vernetzungstyp* | a | a | k | k | k |
| Mischaggregat | Dissolver | Dissolver | Ultra-Turrax | Ultra-Turrax | Ultra-Turrax |
| Drehzahl | 6000 | 6000 | 10000 | 10000 | 10000 |
| Menge Siliconölkomponente in Schritt 1 (g) | 320 | 320 | 170 | 170 | 170 |
| Menge Kieselsäuredispersion aus Beispiel 1 in Schritt 1(g) | 240 | 180 | 130 | 130 | 130 |
| Menge Wasser in Schritt 1 (g) | 29 | - | - | - | - |
| Herstellung Pickering Emulsion gemäß Beispiel | 3 | 3 | 2 | 2 | 4 |
| Herstellung Partikel gemäß Beispiel | 6 | 6 | 5 | 5 | 5 |

(fortgesetzt)

| Beispiel | 16 | 17 | 18 | 19 | 20 |
|---|---|---|---|---|---|
| Q1 | 15, 0 | 10,1 | 15, 3 | 15, 3 | 15, 3 |
| Q2 | 59, 2 | 68, 4 | 62, 0 | 62, 0 | 62, 0 |
| Q3 | 82,6 | 84,2 | 85,9 | 85,9 | 85,9 |
| d50 / μm | 7,6 | 6,1 | 2,2 | 3,2 | 2,2 |
| Verteilungsbreite (d90-d10) | 15, 4 | 8, 6 | 4, 0 | 3, 9 | 3, 8 |
| * Vernetzungstyp: a = additionsvernetzend; k = kondensationsvernetzend | | | | | |

Tabelle 2 (nicht-erfindungsgemäße Beispiele)

| Beispiel | V1 | V2 | V3 | V4 | V5 |
|---|---|---|---|---|---|
| Verwendete Siliconölkomponente | B7 | B7 | B7 | B7 | B8 |
| Viskosität Siliconölkomponente (mPa*s) | 3500 | 3500 | 3500 | 3500 | 30 |
| Vernetzungs typ* | a | a | a | a | k |
| Mischaggreg at | Dissol ver | Dissol ver | Dissol ver | Dissol ver | UltraTur rax |
| Drehzahl | 6000 | 6000 | 6000 | 6000 | 10000 |
| Menge Siliconölko m-ponente in Schritt 1 (g) | 314 | 335 | 297 | 295 | 300 |
| Menge Kieselsäure -dispersion aus Beispiel 1 in Schritt 1 (g) | 160 | 210 | 223 | 146 | 100 |
| Menge Wasser in Schritt 1 (g) | - | 12 | 40 | 49 | - |
| Herstellung Pickering Emulsion gemäß Beispiel | 3 | 3 | 3 | 3 | 2 |
| Herstellung Partikel gemäß Beispiel | - | - | 6 | 6 | 5 |
| Q1 | 11,7 | 15,0 | 12,0 | 7,5 | 6, 7 |
| Q2 | 71,8 | 66,1 | 57,6 | 64,0 | 79,0 |
| Q3 | 90,1 | 89, 5 | 76, 3 | 75,7 | 89, 5 |
| $d_{50}$ / μm | - | - | 60,0 | 62,5 | 38, 1 |
| Verteilungsbreite ($d_{90}$-$d_{10}$) | - | - | 129,0 | 110,6 | 66,1 |
| Bildung Pickering Emulsion | nein | nein | ja | ja | ja |
| * Vernetzungstyp: a = additionsvernetzend; k = kondensationsvernetzend | | | | | |

**Vergleichsbeispiel V6: Herstellung eines nicht-erfindungsgemäßen Partikels gemäß WO07113095**

Ein nicht-erfindungsgemäßer Silica-beschichteter SiliconharzPartikel wurde gemäß WO07113095 Beispiel 1a) bis 1c) hergestellt. Dabei entspricht Q1 = 7,7, Q2 = 79,5 und Q3 = 91,5. Es wird ein Feststoff mit einer mittleren Partikelgröße d50 = 9,3 μm und eine Verteilungsbreite (d90 - d10) = 27,2 erhalten.

**Anwendungsbeispiele**

**Beispiel 21: Sensorische Bewertung in kosmetischer Anwendung**

Die Evaluierung der sensorischen Eigenschaft der erfindungsgemäßen feinteiligen Silica-beschichteten Siliconpartikel aus den Beispielen 13 und 18 und der nicht-erfindungsgemäßen Silica-beschichteten Siliconpartikel aus den Vergleichsbeispielen V3 und V5 erfolgte durch eine geschulte Gruppe von Probanden. Nach dem Auftragen auf die Haut wurden die sensorischen Eigenschaften der Rückstände relativ zueinander bewertet. Tabelle 3 zeigt die durchschnittliche Bewertung der Probanden, wobei die Note 5 einem bevorzugten samtig-seidenen Hautgefühl entspricht und die Note 0 einem unerwünschten kratzig-rauen Hautgefühl entspricht.

TABELLE 3 - BEWERTUNG DER SENSORISCHE EIGENSCHAFTEN:

| Organopolysiloxangel | Note (Skala 0 bis 5; 5 = bestmögliche Bewertung) |
|---|---|
| Beispiel 13 | 1 |
| Beispiel 18 | 2 |
| Vergleichsbeispiel V3* | 3 |
| Vergleichsbeispiel V5* | 4 |
| *nicht erfindungsgemäß | |

**Beispiel 22: Anwendung in Beschichtungen**

Eine Siliconbeschichtung wurde hergestellt. Dazu wurden 2 Teile der erfindungsgemäßen feinteiligen Silica-beschichteten Siliconpartikeln aus dem Beispiel 18 mit 98 Teile der Siliconmasse B7 homogen vermischt, indem 10 min mit einem Dissolver bei 6000 upm gerührt wurde, wobei die Temperatur auf 20 °C gehalten wurde. Die resultierende Masse wurde mit einem 10 $\mu$m Rakel auf eine Glasplatte aufgebracht. Man erhält eine transparente glatte Beschichtung.

**Vergleichsbeispiel V7: Anwendung in Beschichtungen**

Im Unterschied zu Beilspiel 22 wurden die nicht-erfindungsgemäßen Silica-beschichteten Siliconpartikel aus Vergleichsbeispiel V5 verwendet. Die resultierende Beschichtung ist inhomogen und weist zahlreiche weiße Körner und Streifen auf.

**Beispiel 23: Optische Bewertung in kosmetischer Anwendung**

100 mg der erfindungsgemäßen feinteiligen Silica-beschichteten Siliconpartikel aus dem Beispiel 13 wurden gleichmäßig auf eine kreisrunde Fläche mit 4 cm Durchmesser auf dem ungereinigten Unterarm eines Probanden verteilt. Man erhält eine trockene, gleichmäßige, optisch homogene und leicht weißliche Hautfläche. weißliche Hautfläche. Dies ist ein Zeichen, dass vorhandenes Sebum von der Hautoberfläche vollständig adsorbiert wird.

**Vergleichsbeispiel V8: Optische Bewertung in kosmetischer Anwendung**

Im Unterschied zu Beispiel 23 wurden Siliconpartikel nach dem Stand der Technik (Tospearl 2000B Mikrokügelchen erhältlich bei Momentive Performance Materials) verwendet. Die Partikel klumpen beim Verteilen zusammen und ergeben ein unschönes, inhomogenes Hautbild. Dies ist ein Zeichen, dass vorhandenes Sebum von der Hautoberfläche nicht vollständig adsorbiert werden kann.

**Patentansprüche**

1.   Verfahren zur Herstellung einer wässrigen partikelstabilisierten Pickering Emulsion (E) eines polyadditionsfähigen, polykondensationsfähigen oder polymerisierbaren Materials (S), das ausgewählt wird aus Siloxan und Silan,

   bei dem eine wässrige Phase (W),
   ein polyadditionsfähiges, polykondensationsfähiges oder polymerisierbares Material (S), das ausgewählt wird aus Siloxan und Silan und
   ein partikulärer Silicium(IV)oxid Feststoff (F) vermischt werden,
   wobei Tröpfchen mit einem mittleren Durchmesser d50 von höchstens 9 $\mu$m gebildet werden, welche das Material (S) und den partikulären Feststoff (F) umfassen,
   mit der Maßgabe, dass in der partikelstabilisierten Pickering-Emulsion
   das Massenverhältnis Q1 = m(F)/m(S)*100 eine beliebige Zahl von 3 bis 25 ist,
   das Massenverhältnis Q2 = m(S)/(m(S)+m(W))*100 eine beliebige Zahl von 50 bis 75 ist und

Q1 und Q2 zueinander im Zusammenhang

$$Q2 = -(1,56 * Q1) + Q3$$

stehen, wobei
Q3 eine Zahl zwischen 77,0 und 89,0 ist,
wobei die Bestimmung des $d_{50}$-Werts mit einem Camsizer X2 von Retsch Technology erfolgt (Messprinzip: Dynamische Bildanalyse nach ISO 13322-2, Messbereich: 0,8 $\mu$m - 30 mm, Art der Analyse: Trockenmessung von Pulvern und Granulaten, Dispergierdruck = 2 bar).

2. Verfahren nach Anspruch 1, bei dem das Material (S) zumindest ein polyadditionsfähiges, polykondensationsfähiges oder polymerisierbares Siloxan der allgemeinen Formel (IV)

$$[A^1_m R^9_p SiO_{(4-p-m)/2}] \qquad (IV),$$

ist, wobei

$A^1$ einen Wasserstoff- oder Kohlenwasserstoffrest bedeutet, der bis zu 30 C-Atome enthält und zusätzlich Heteroatome ausgewählt aus O-, S, Si, Cl, F, Br, P oder N-Atomen enthalten kann, so dass $A^1$ auch eine funktionelle Gruppe bedeuten kann, die selbst unsubstituiert oder substituiert ist,
$R^9$ Alkoxy- oder Aryloxyreste mit bis zu 18 C-Atomen, Hydroxyreste oder H bedeutet, oder die unabhängig von $A^1$ dessen Bedeutung haben kann,
$m$ und $p$ jeweils die Werte 0, 1, 2 oder 3 bedeuten oder zumindest ein polyadditionsfähiges, polykondensationsfähiges oder polymerisierbares Silan der allgemeinen Formel (V),

$$(R^{10})_{4-n}\text{-Si-}(OR^{11})_o \qquad (V)$$

wobei $o$ eine Zahl im Wert von 1, 2, 3 oder 4 bedeutet,

$R^{10}$ lineare oder verzweigte Alkylreste mit 1 bis 16 Kohlenstoffatomen, wobei nicht benachbarte Kohlenstoffatome durch Sauerstoffatome ersetzt sein können oder Arylreste bedeutet oder ein organofunktioneller Rest ist, ausgewählt aus der Gruppe Phosphonsäuremonoesterrest, Phosphonsäurediesterrest, Phosphonsäurerest, Methacryloyloxyrest, Acryloyloxyrest, Vinylrest, Mercaptorest, Isocyanatorest, wobei der Isocyanatorest gegebenenfalls zum Schutze vor chemischen Reaktionen reaktionsblockiert sein kann, Hydroxyrest, Hydroxyalkyrest, Vinylrest, Epoxyrest, Glycidyloxyrest, Morpholinorest, Piperazinorest, einen primären, sekundären oder tertiären Aminorest mit einem oder mehreren Stickstoffatomen, wobei die Stickstoffatome durch Wasserstoff oder einwertige aromatische, aliphatische oder cycloaliphatische Kohlenwasserstoffreste substituiert sein können, Carbonsäurerest, Carbonsäureanhydridrest, Aldehydrest, Urethanrest, Harnstoffrest, wobei der Rest $R^{10}$ unmittelbar am Siliziumatom gebunden sein oder durch eine Kohlenstoffkette von 1 - 6 C-Atomen davon getrennt sein kann, und
$R^{11}$ einen einwertigen linearen oder verzweigten aliphatischen oder cycloaliphatischen Kohlenwasserstoffrest, in dem nicht benachbarte Kohlenstoffatome durch Heteroatome wie O, N, P, S, Cl, F, Br oder Si ersetzt sein können, wobei die freien Valenzen der betreffenden Heteroatome durch lineare oder verzweigte Alkylreste oder durch Wasserstoffatome abgesättigt sein können oder einen einwertigen aromatischen Kohlenwasserstoffrest oder einen Rest der Form -C(=O)-$R^{12}$ bedeutet, wobei $R^{12}$ einen einwertigen linearen oder verzweigten aliphatischen oder einen cycloaliphatischen Kohlenwasserstoffrest oder einen einwertigen aromatischen Kohlenwasserstoffrest bedeutet, wobei das ausgewählte Silan oder gegebenenfalls die ausgewählten Silane in nicht hydrolysierter Form, in hydrolysierter Form oder in hydrolysierter und teilkondensierter oder hydrolysierter und kondensierter Form oder in einem Gemisch dieser Formen vorliegen können,

oder eine Zubereitung mehrerer solcher Siloxane der allgemeinen Formel (IV) und / oder Silane der allgemeinen Formel (V).

3. Verfahren nach einem oder mehreren der vorangehenden Ansprüche, bei dem der partikuläre Feststoff (F) eine Löslichkeit in Wasser bei pH 7,33 und einem Elektrolythintergrund von 0,11 Mol, einer Temperatur von 37°C und bei 1013 hPa von kleiner 0,1 g/l aufweist.

4. Verfahren nach einem oder mehreren der vorangehenden Ansprüche, bei dem die mittlere Partikelgröße des partikulären Feststoffs (F) kleiner als 1000 nm ist.

5. Verfahren nach einem oder mehreren der vorangehenden Ansprüche, bei dem der Kohlenstoffgehalt des partikulären Feststoffs (F) 0,1 - 4 Gew.% beträgt, gemessen mittels Elementaranalyse an den trockenen partikulären Feststoffen.

6. Wässrige partikelstabilisierte Pickering Emulsion (E) herstellbar gemäß dem Verfahren nach einem oder mehreren der vorangehenden Ansprüche.

7. Verfahren zur Herstellung von Partikeln (P) aus einem Kern (K) enthaltend polymeres Material und einer Hülle (H) aus partikulärem Feststoff (F),

bei dem in einem ersten Schritt eine wässrige partikelstabilisierten Pickering Emulsion (E) eines polyadditionsfähigen, polykondensationsfähigen oder polymerisierbaren Materials (S), das ausgewählt wird aus Siloxan und Silan, gemäß einem oder mehreren der Ansprüche 1 bis 5 hergestellt wird und
in einem zweiten Schritt das Material (S) der wässrigen partikelstabilisierten Pickering Emulsion (E) einer Polyaddition, Polymerisation oder Kondensation unterzogen wird.

8. Verfahren nach Anspruch 7, bei dem die Partikel (P) eine Verteilungsbreite ($d_{90}$-$d_{10}$) kleiner als 20 aufweisen.

9. Additionsvernetzte, kondensationsvernetzte oder polymerisationsvernetzte Siliconharzpartikel (P1) mit einem mittleren Durchmesser d50 von höchstens 9 $\mu$m, herstellbar nach dem vorstehend beschriebenen Verfahren.


**Claims**

1. Process for producing an aqueous, particle-stabilized Pickering emulsion (E) of a material (S) amenable to polyaddition, to polycondensation or to chain polymerization and selected from siloxane and silane,

by mixing an aqueous phase (W),
a material (S) amenable to polyaddition, to polycondensation or to chain polymerization and selected from siloxane and silane, and
a particulate silicon(IV) oxide solid (F),
forming droplets having a mean diameter d50 of at most 9 $\mu$m and comprising the material (S) and the particulate solid (F),
with the proviso that in the particle-stabilized Pickering emulsion
the mass ratio Q1 = m(F)/m(S)*100 is any number from 3 to 25,
the mass ratio Q2 = m(S)/(m(S)+m(W))*100 is any number from 50 to 75, and
the relationship between Q1 and Q2 is

$$Q2 = -(1.56 *Q1) + Q3,$$

where
Q3 is a number between 77.0 and 89.0,
where the $d_{50}$ is determined using a Camsizer X2 from Retsch Technology (measurement principle: dynamic image analysis according to ISO 13322-2, measurement range: 0.8 um - 30 mm, type of analysis: dry measurement of powders and granules, dispersing pressure = 2 bar).

2. Process according to Claim 1, wherein the material (S) is at least one siloxane amenable to polyaddition, to polycondensation or to chain polymerization and of the general formula (IV)

$$[A^1{}_mR^9{}_pSiO_{(4-p-m)/2}] \qquad (IV),$$

where

$A^1$ is a hydrogen or hydrocarbon radical which contains up to 30 carbon atoms and may additionally contain heteroatoms selected from O, S, Si, Cl, F, Br, P or N atoms, and so $A^1$ may also be a functional group, which itself is unsubstituted or substituted,

$R^9$ denotes alkoxy or aryloxy radicals having up to 18 carbon atoms, or hydroxyl radicals or H, or which independently of $A^1$ may have the definition thereof,

$m$ and $p$ each denote the values 0, 1, 2 or 3,

or at least one silane amenable to polyaddition, to polycondensation or to chain polymerization and of the general formula (V)

$$(R^{10})_{4-n}\text{-Si-}(OR^{11})_o \qquad (V),$$

where $o$ is a number with a value of 1, 2, 3 or 4,

$R^{10}$ denotes linear or branched alkyl radicals having 1 to 16 carbon atoms, where nonadjacent carbon atoms may be replaced by oxygen atoms, or denotes aryl radicals or is an organofunctional radical selected from the group of phosphonic monoester radical, phosphonic diester radical, phosphonic acid radical, methacryloyloxy radical, acryloyloxy radical, vinyl radical, mercapto radical, isocyanato radical, where the isocyanato radical may optionally be reaction-blocked for protection from chemical reactions, hydroxyl radical, hydroxyalkyl radical, vinyl radical, epoxy radical, glycidyloxy radical, morpholino radical, piperazino radical, a primary, secondary or tertiary amino radical having one or more nitrogen atoms, where the nitrogen atoms may be substituted by hydrogen or by monovalent aromatic, aliphatic or cycloaliphatic hydrocarbon radicals, carboxylic acid radical, carboxylic anhydride radical, aldehyde radical, urethane radical, urea radical, where the radical $R^{10}$ may be bonded directly on the silicon atom or may be separated therefrom by a carbon chain of 1 - 6 carbon atoms, and $R^{11}$ is a monovalent, linear or branched aliphatic or cycloaliphatic hydrocarbon radical in which nonadjacent carbon atoms may be replaced by heteroatoms such as O, N, P, S, Cl, F, Br or Si, where the free valences of the relevant heteroatoms may be satisfied by linear or branched alkyl radicals or by hydrogen atoms, or is a monovalent, aromatic hydrocarbon radical or a radical of the form -C(=O)-$R^{12}$, where $R^{12}$ is a monovalent, linear or branched aliphatic or a cycloaliphatic hydrocarbon radical or a monovalent, aromatic hydrocarbon radical, where the selected silane or optionally the selected silanes may be present in nonhydrolyzed form, in hydrolyzed form, or in hydrolyzed and partly condensed or hydrolyzed and condensed form, or in a mixture of these forms, or a preparation of two or more such siloxanes of the general formula (IV) and/or silanes of the general formula (V).

3. Process according to one or more of the preceding claims, wherein the particulate solid (F) has a solubility in water at pH 7.33 and an electrolyte background of 0.11 mol, a temperature of 37°C and at 1013 hPa of less than 0.1 g/l.

4. Process according to one or more of the preceding claims, wherein the mean particle size of the particulate solid (F) is less than 1000 nm.

5. Process according to one or more of the preceding claims, wherein the carbon content of the particulate solid (F) is 0.1 - 4 wt%, measured by means of elemental analysis on the dry particulate solids.

6. Aqueous, particle-stabilized Pickering emulsion (E) producible by the process according to one or more of the preceding claims.

7. Process for producing particles (P) comprising a core (K) containing polymeric material and a shell (H) composed of particulate solid (F), wherein, in a first step, an aqueous, particle-stabilized Pickering emulsion (E) of a material (S) amenable to polyaddition, to polycondensation or to chain polymerization and selected from siloxane and silane is produced according to one or more of Claims 1 to 5, and in a second step, the material (S) of the aqueous, particle-stabilized Pickering emulsion (E) is subjected to a polyaddition, chain polymerization or condensation.

8. Process according to Claim 7, wherein the particles (P) have a distribution range ($d_{90}$-$d_{10}$) of less than 20.

9. Addition-crosslinked, condensation-crosslinked or chain polymerization-crosslinked silicone resin particles (P1) having a mean diameter d50 of at most 9 $\mu$m, producible by the process described above.

**Revendications**

1. Procédé pour la préparation d'une émulsion de Pickering (E) aqueuse à particules stabilisées d'un matériau (S) apte à une polyaddition, à une polycondensation ou polymérisable, qui est choisi parmi un siloxane et un silane,

   dans lequel une phase aqueuse (W),
   un matériau (S) apte à une polyaddition, à une polycondensation ou polymérisable, qui est choisi parmi un siloxane et un silane et
   un solide particulaire (F) d'oxyde de silicium (IV) sont mélangés,
   des gouttelettes présentant un diamètre moyen $d_{50}$ d'au plus 9 μm étant formées, qui comprennent le matériau (S) et le solide particulaire (F), à condition que dans l'émulsion de Pickering à particules stabilisées le rapport massique Q1 = m(F)/m(S)*100 soit un nombre quelconque de 3 à 25,
   le rapport massique Q2 = m(S) / (m(S) +m(W)) *100 soit un nombre quelconque de 50 à 75 et
   Q1 et Q2 soient liés par la relation

$$Q2 = -(1,56 *Q1) + Q3$$

   où
   Q3 représente un nombre entre 77,0 et 89,0,
   la détermination de la valeur $d_{50}$ ayant lieu à l'aide d'un Camsizer X2 de la société Retsch Technology (principe de mesure : analyse dynamique d'image selon la norme ISO 13322-2, plage de mesure : 0,8 μm-30 mm, type d'analyse : mesure à sec de poudres et de granulats, pression de dispersion = 2 bars).

2. Procédé selon la revendication 1, dans lequel le matériau (S) est au moins un siloxane apte à une polyaddition, à une polycondensation ou polymérisable de formule générale (IV)

$$[A^1{}_m R^9{}_p SiO_{(4-p-m)/2}] \qquad (IV),$$

   où

   $A^1$ signifie un radical d'hydrogène ou hydrocarboné, qui contient jusqu'à 30 atomes de carbone et qui peut en outre contenir des hétéroatomes choisis parmi les atomes d'O, de S, de Si, de Cl, de F, de Br, de P ou de N, de manière telle que $A^1$ peut également signifier un groupe fonctionnel, qui est lui-même non substitué ou substitué,
   $R^9$ signifie des radicaux alcoxy ou aryloxy comprenant jusqu'à 18 atomes de carbone, des radicaux hydroxy ou H ou peut présenter la signification de $A^1$, indépendamment de celui-ci,
   m et p signifient à chaque fois les valeurs 0, 1, 2 ou 3
   ou au moins un silane apte à une polyaddition, à une polycondensation ou polymérisable de formule générale (V)

$$(R^{10})_{4-n}\text{-Si-}(OR^{11})_o \qquad (V)$$

   dans laquelle

   o signifie un nombre de valeur 1, 2, 3 ou 4,
   $R^{10}$ signifie des radicaux alkyle linéaires ou ramifiés comprenant 1 à 16 atomes de carbone, des atomes de carbone non adjacents pouvant être remplacés par des atomes d'oxygène ou signifie des radicaux aryle ou est un radical organofonctionnel, choisi dans le groupe radical monoester d'acide phosphonique, radical diester d'acide phosphonique, radical d'acide phosphonique, radical méthacryloyloxy, radical acryloyloxy, radical vinyle, radical mercapto, radical isocyanato, le radical isocyanato pouvant le cas échéant être bloqué en réaction pour la protection contre des réactions chimiques, radical hydroxy, radical hydroxyalkyle, radical vinyle, radical époxy, radical glycidyloxy, radical morpholino, radical pipérazino, un radical amino primaire, secondaire ou tertiaire comprenant un ou plusieurs atomes d'azote, les atomes d'azote pouvant être substitués par hydrogène ou par des radicaux hydrocarbonés monovalents aromatiques, aliphatiques ou cycloaliphatiques, radical acide carboxylique, radical anhydride d'acide carboxylique, radical aldéhyde, radical uréthane, radical urée, le radical $R^{10}$ pouvant être lié directement à l'atome de silicium ou séparé de celui-ci par une chaîne carbonée de 1-6 atomes de carbone et

R[11] signifie un radical hydrocarboné monovalent aliphatique linéaire ou ramifié ou cycloaliphatique, dans lequel des atomes de carbone non adjacents peuvent être remplacés par des hétéroatomes tels que O, N, P, S, Cl, F, Br ou Si, les valences libres des hétéroatomes concernés pouvant être saturées par des radicaux alkyle linéaires ou ramifiés ou par des atomes d'hydrogène ou signifie un radical hydrocarboné monovalent aromatique ou un radical de formule -C(=O)-R[12], dans laquelle R[12] signifie un radical hydrocarboné monovalent, aliphatique linéaire ou ramifié ou cycloaliphatique ou un radical hydrocarboné monovalent aromatique, le silane choisi ou le cas échéant les silanes choisis pouvant se trouver sous forme non hydrolysée, sous forme hydrolysée ou sous forme hydrolysée et partiellement condensée ou sous forme hydrolysée et condensée ou sous forme de mélange de ces formes,

ou une préparation de plusieurs de ces siloxanes de formule générale (IV) et/ou silanes de formule générale (V) .

3. Procédé selon l'une ou plusieurs des revendications précédentes, dans lequel le solide particulaire (F) présente une solubilité dans l'eau, à pH 7,33 et dans un environnement électrolytique de 0,11 mole, à une température de 37°C et à 1013 hPa, inférieure à 0,1 g/l.

4. Procédé selon l'une ou plusieurs des revendications précédentes, dans lequel la grosseur moyenne de particule du solide particulaire (F) est inférieure à 1000 nm.

5. Procédé selon l'une ou plusieurs des revendications précédentes, dans lequel la teneur en carbone du solide particulaire (F) est de 0,1-4% en poids, mesurée par analyse élémentaire sur les solides particulaires secs.

6. Émulsion de Pickering (E) aqueuse à particules stabilisées, pouvant être préparée selon le procédé selon l'une ou plusieurs des revendications précédentes.

7. Procédé pour la préparation de particules (P) constituées par un noyau (K) contenant un matériau polymère et une enveloppe (H) constituée par un solide particulaire (F),

   dans lequel, dans une première étape, une émulsion de Pickering (E) aqueuse à particules stabilisées d'un matériau (S) apte à une polyaddition, à une polycondensation ou polymérisable, qui est choisi parmi un siloxane et un silane, est préparée selon l'une ou plusieurs des revendications 1 à 5, et
   dans une deuxième étape, le matériau (S) de l'émulsion de Pickering (E) aqueuse, à particules stabilisées est soumise à une polyaddition, à une polymérisation ou à une condensation.

8. Procédé selon la revendication 7, dans lequel les particules (P) présentent une largeur de répartition ($d_{90}$-$d_{10}$) inférieure à 20.

9. Particules de résine de silicone (P1) réticulées par addition, par condensation ou par polymérisation, présentant un diamètre moyen $d_{50}$ d'au plus 9 mm, pouvant être préparées selon le procédé décrit ci-dessus.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2017142068 A1
- WO 2007113095 A
- EP 433727 A
- DE 102006014875
- DE 102015216415
- EP 1433749 A1
- DE 10349082 A1
- WO 07113095 A

**In der Beschreibung aufgeführte Nicht-Patentliteratur**

- **G. LAGALY ; O. SCHULZ ; R. ZINDEL.** *Steinkopff,* 1997, ISBN 3-7985-1087-3, 1-4